**Europäisches Patentamt**

**European Patent Office**

**Office européen des brevets**

(11) Veröffentlichungsnummer: **0 429 393 A2**

(12) **EUROPÄISCHE PATENTANMELDUNG**

(21) Anmeldenummer: **90810847.5**

(22) Anmeldetag: **06.11.90**

(51) Int. Cl.5: **B31D 1/04**

(30) Priorität: **17.11.89 CH 4151/89**

(43) Veröffentlichungstag der Anmeldung:
**29.05.91 Patentblatt 91/22**

(84) Benannte Vertragsstaaten:
**CH DE FR GB IT LI**

(71) Anmelder: **PRO.GE.SAN. SRL**
**Via Cortina d'Ampezzo, 186**
**I-00135 Rom(IT)**

(72) Erfinder: **Rossi,Guido**
**Wickenweg 19**
**CH-8048 Zürich(CH)**

(74) Vertreter: **Breiter, Heinz et al**
**Patentanwalt H. Breiter AG**
**Schaffhauserstrasse 27 Postfach 1163**
**CH-8401 Winterthur(CH)**

(54) **Vorrichtung zur Formung von Saugkissen zum Aufsaugen von Körperflüssigkeit in Hygieneartikeln.**

(57) Bei einer Vorrichtung zur körpergerechten Formung von Saugkissen für Baby- und Erwachsenen-Windeln, Damenbinden, Slipeinlagen u.dgl. bildet eine einzige vorzugsweise aus Zellstoff/Synthetik-Pulpe bestehende Schicht kontinuierlich das Profil des Saugkissens sowohl in Quer- als auch in Längsrichtung. Zur Bildung der Saugkissen ist ein endloses über eine Antriebswalze (20), einen Vakuumtisch (18), eine Walze (21) und eine einstellbare Spannwalze (22) geführtes Siebband (23) vorgesehen, wobei oberhalb des Vakuumtisches (18) in einem Gehäuse (11) mindestens eine Kammwalze (14) mit je einer über deren Breite unterschiedlich hohen Benadelung (14′) angeordnet ist und das Profil des Saugkissen in Querrichtung ausbildet. Nachfolgend ist eine vertikal bewegbare Hubkammwalze (15) mit einer über deren Breite gleich hohen Benadelung (15′) angeordnet, die das Profil des Saugkissen in Längsrichtung ausbildet. Zum Verfestigen gelangen derartig geformte Saugkissen über eine Rutsche (29) zu einem Anpress-Walzenpaar (27,28), wobei die obere Anpresswalze (28) aus mehreren, auf einer Welle frei unabhängig voneinander drehenden Scheibenwalzen (28′) und einer unteren glatt ausgebildeten Anpresswalze (27) gebildet ist.

FIG. 1

## VORRICHTUNG ZUR FORMUNG VON SAUGKISSEN ZUM AUFSAUGEN VON KÖRPERFLÜSSIGKEIT IN HYGIENEARTIKELN

Die Erfindung betrifft eine Vorrichtung zur Formung von Saugkissen zum Aufsaugen von Körperflüssigkeit in Hygieneartikeln, gemäss dem Oberbegriff des Patentanspruchs 1.

Es sind Vorrichtungen zur Herstellung von Saugkissen bekannt, bei welchen mittels mindestens einer Kammwalze ein saugkissenartige Materialbahn kontinuierlich gelegt wird. Um das Profil der Saugkissen insbesondere im Bereich der stärker anfallenden Körperflüssigkeit zu vergrössern, wurde gemäss der deutschen Patentschrift DE 34 13 925 eine zweite Saugkissen-Teilschicht auf die erste aufgebracht. Die Vorrichtung besteht aus zwei gleichartig ausgebildeten, sogenannten Flockenlegern, wobei jeder der beiden Flockenleger als ein Saugzylinder ausgebildet ist, der Formvertiefungen aufweist und das sogenannte Flockenrad bildet. Auf dem ersten Flockenrad wird eine erste Teilschicht und auf dem zweiten Flockenrad wird eine zweite Teilschicht des zu fertigenden Saugkissens angesaugt. Mittels einer Uebergabewalze werden die auf dem ersten Flockenrad gefertigten Teilschichten auf die auf dem zweiten Flockenrad gefertigten Teilschichten aufgebracht. Ein weiterer Uebergabezylinder hebt die fertigen Profil-Saugkissen vom zweiten Flockenrad ab und überführt sie mittels eines Sauggurtes zu einer nachfolgenden Fertigungsmaschine.

Das Umrüsten derartiger Vorrichtungen ist sehr zeitaufwendig, so dass sich diese nur als Einzweckmaschinen eignen.

Ferner sind Anordnungen bekannt, bei welchen zur Bildung des Saugkissen-Profiles in Querrichtung eine Kammwalze mit einer ein Profil bildenden Benadelung vorgesehen ist, wobei ein derartiges, in Querrichtung gebildetes Saugkissen-Profil über die gesamte Länge der endlos miteinander verbundenen Saugkissen verläuft. Um die Saugkissen auch in Längsrichtung auszubilden, werden die Bereiche ausserhalb des eigentlichen Profils in Verfestigungseinrichtungen zusammengepresst.

Hierdurch ergeben sich jedoch entscheidende Nachteile, indem die in Längsrichtung gepressten Bereiche beim Tragen sich unangenehm als zu hart erweisen und eine verminderte Saugfähigkeit aufweisen. Dadurch, dass in den ausserhalb des Saugkissen-Profils liegenden Bereichen in unerwünschter Weise mehr Material als notwendig verbraucht wird, entstehen konstenaufwendige Materialverluste an Zellstoff/Synthetik-Pulpe (Flockenmaterial).

Ausgehend von diesem Stand der Technik ist es Aufgabe der Erfindung, eine Anordnung zu schafffen, welche auf eine einfache Weise und bei optimalem Materialverbrauch sowohl in Quer- als auch in Längsrichtung beliebig geformte Saugkissen, ohne zeitaufwendige Schichtung von Teilschichten, ermöglicht.

Die Aufgabe wird erfindungsgemäss durch die im kennzeichnenden Teil des Anspruchs 1 angegebenen Merkmale gelöst, während die übrigen Ansprüche vorteilhafte weitere Ausgestaltungen der Vorrichtung betreffen.

Der Vorteil der Erfindung besteht insbesondere darin, dass die Formung des Saugkissens bei der Bildung einer einzigen Schicht das Profil sowohl in Querrichtung als auch in Längsrichtung der kontinuierlich hergestellten Saugkissen erfolgt, wodurch die Produktionskapazität gegenüber bisher mehrschichtigen Saugkissen wesentlich erhöht werden kann.

Durch Hubsteuerung der Hubkammwalze ggf. zusammen mit der Hub-Profilwalze für das Siebband kann das in Längsrichtung für ein beliebiges Saugkissen-Profil überschüssige Material abgetragen werden, wobei über in der Material-Zuführleitung geführte Rückleitungen das zurückgeführte Material dem neu zugeführten Material beigemischt wird, so dass kein Materialabfall entsteht.

Zur Bildung des Saugkissen-Profils in Längsrichtung kommt der Einfachheit halber besonders vorteilhaft einzig die vertikal bewegbaren Hubkammwalze zur Anwendung.

In einer weiteren vorteilhaften Ausbildung der Vorrichtung kann zur Hubkammwalze zusätzlich noch zum Anheben des Siebbandes eine Profil-Hubwalze angeordnet sein, wobei das angehobene Siebband in den Profilbereich der Benadelung hineinragt, und die Hubkammwalze in einer vorbestimmten Position fixierbar oder anheb- und absenkbar ausgebildet ist, so dass je nach Bedarf verschiedene Saugkissen-Profile herstellbar sind.

Ein weiterer Vorteil besteht in der Anordnung von Rollen bzw. Kugeln am Vakuumtisch, wodurch die Reibung -auch bei sehr hohen Vakuum- zwischen dem Siebband und dem Vakuumtisch minimal gehalten werden kann und dadurch die Lebensdauer des Siebbandes wesentlich verlängert werden kann.

Besonders vorteilhaft ist, dass die erfindungsgemässen Vorrichtungen ohne grossen Aufwand in bereits bestehende Fertigungsanlagen integrierbar sind.

In der Zeichnung sind zwei Ausfürhungsbeispiele des Erfindungsgegenstandes schematisch dargestellt.

Es zeigt

Fig.1 einen Längsschnitt der Vorrichtung,

Fig.1a einen Querschnitt durch eine Kammwalze entlang der Linie A-A in Fig.1

Fig.1b einen Querschnitt einer Hubkammwalze entlang der Linie B-B in Fig.1

Fig.2 einen Längsschnitt einer weiteren Ausbildung der in Fig.1 dargestellten Vorrichtung,

Fig.2a einen Querschnitt einer Kammwalze entlang der Linie C-C in Fig.2,

Fig.2b einen Querschnitt einer Hubkammwalze und einer Hub-Profilwalze mit Siebband entlang der Linie D-D in Fig.2,

Fig.2c einen Querschnitt eines Anpress-Walzenpaares entlang der Linie E-E in Fig.2,

Fig.2d eine perspektivische Darstellung von in Querrichtung und Längsrichtung geformten Saugkissen, und

Fig.2e einen Teil-Längsschnitt des mit Rollenwalzen und Vakuumschlitzen ausgebildeten Vakuumtisches gemäss der Fig.1 und 2.

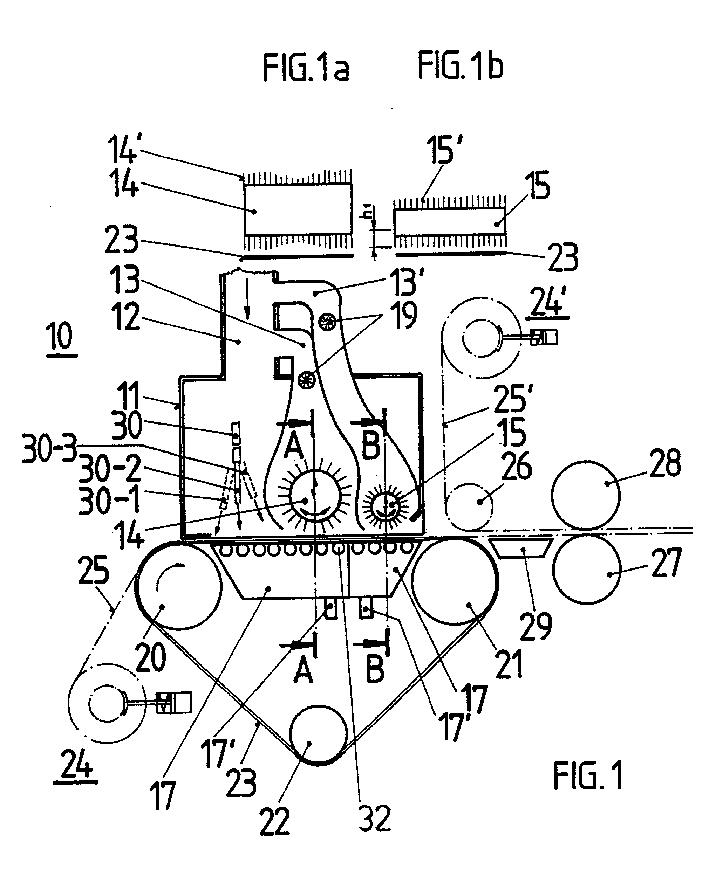

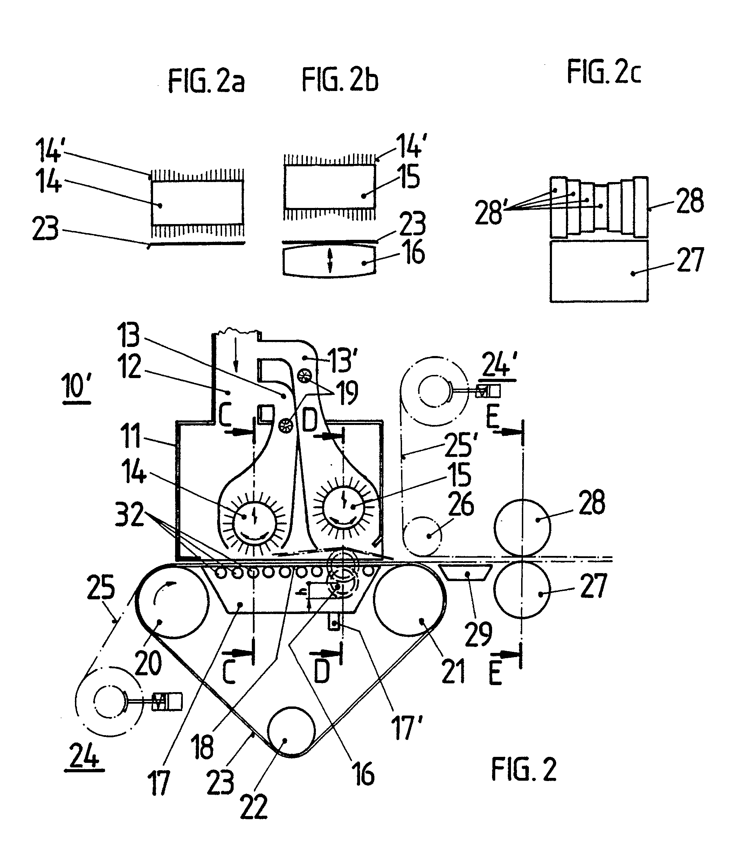

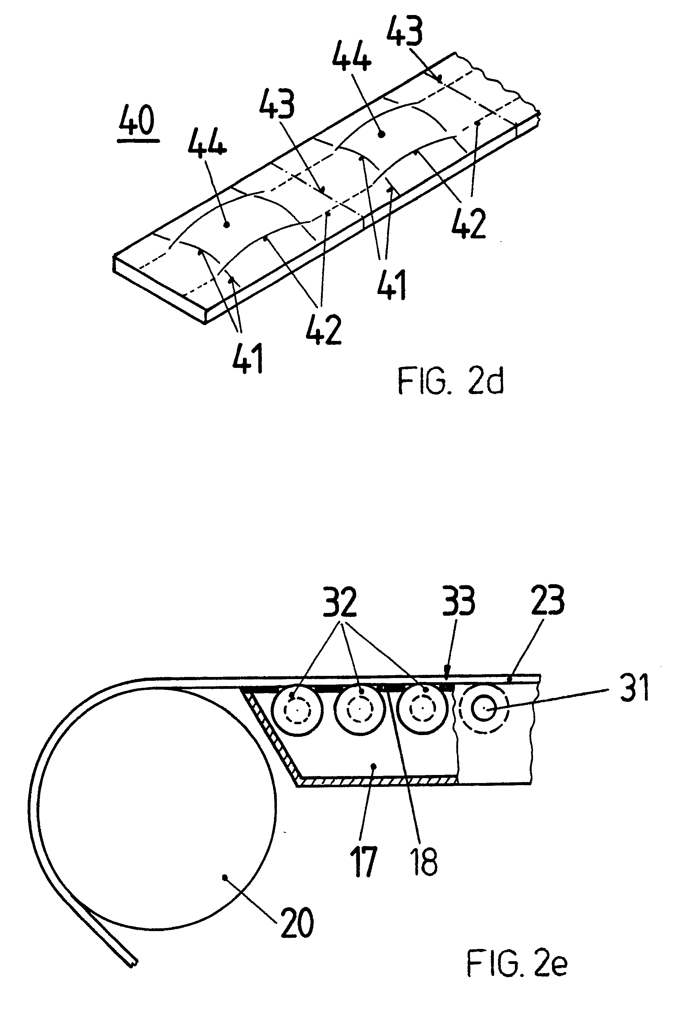

Gemäss den Fig.1,1a,1b bzw.2,2a,2b,2c,2d und e2 ist die Vorrichtung jeweils mit den Bezugszeichen 10 bzw. 10′ bezeichnet.Gleiche Teile sind in allen Figuren mit denselben Bezugszeichen versehen.

In den Fig.1 und 2 bestehen die Vorrichtungen 10,10′ aus einem Gehäuse 11, in welches aus einer nicht dargestellten Material-Bereitstellung über eine MaterialZuführungsleitung 12 das Material, vorzugsweise ein bekanntes Zellstoff/Synthetik-Pulpegemisch zugeführt wird. Unterhalb des nach unten offenen Gehäuses 11 ist ein Vakuumtisch 18 angeordnet, der aus dem oberen Teil einer Vakuumkammer 17 gebildet ist. Zwischen dem Vakuumtisch 18 und dem nach unten offenen Gehäuse 11 ist ein endloses Siebband 23 angeordnet, das durch eine Antriebswalze 20 angetrieben und über eine Walze 21 und eine einstellbare Spannwalze 22 geführt wird. Im Vakuumtisch 18 sind Lagerungen 31 (siehe Fig.2e) vorgesehen, in welche beispielsweise Rollenwalzen 32 eingesetzt werden, die geringfügig über die Oberkanten des Vakuumtisches 18 herausragen, wodurch eine störungsfreie Bewegung des Siebbandes 23 gewährleistet wird. Im Gehäuse 11 ist oberhalb des Siebandes 23 eine einstellbare Kammwalze 14 mit einer profilartig ausgebildeten Benadelung 14′ angeordnet. Das in das Gehäuse 11 auf das unter Vakuum stehenden Siebband 23 zugeführte Flockenmaterial wird für das zu bildende Saugkissen durch die Kammwalze 14 zu einer in Querrichtung des Siebbandes 23 profilartigen Form ausgebildet. Mittels einer der Kammwalze 14 vorgeschalteten an sich bekannten Dosiereinrichtung 30 für chemische Absorptionsmittel , kann während der Schichtbildung durch Verstellung der Dosiereinrichtung 30 in die Positionen 30-1,30-2,30-3 beispielsweise ein Superabsorber in vorbestimmte Saugkissen-Bereiche dosiert eingeführt werden.

Nachfolgend wird gemäss Fig.1 erfindungsgemäss durch eine vertikal bewegbare Haubkammwalze 15 mit einer über deren gesamte Breite gleich hohen Benadelung 15′, das in Querrichtung geformte Saugkissen durch Abtragen bzw.Stehenlassen des Materials durch gesteuertes Absenken bzw. Anheben der Hubkammwalze 15 zusätzlich in Längsrichtung des Siebbandes 23 geformt. Das überschüssige Material wird über in die Zuführleitung 12 mündende Rückführungsleitungen 13,13′ mit dem neuen Material gemischt. In den Rückleitungen 13,13′ sind Saugluftventilatoren 19 angeordnet. Zur Luftwirbeldämpfung können die einzelnen Nadeln der Benadelungen 14′,15′ der Kammwalzen und Hubkammwalzen 14,15 verstellbar sein und ein flachpropellartiges Profil aufweisen. Die in Quer- und Längsrichtung geformten Saugkissen können mit von Abwickelvorrichtungen 24 bzw.24′ abgezogenen Abdeck-Papierbahnen 25 bzw.25′, die von unten bzw. von oben über eine Umlenkwalze 26 zugeführt werden, abgedeckt werden.

Zum Verfestigen gelangen die endlos verbundenen Saugkissen 40 über eine Rutsche 29 zu einem Anpress-Walzenpaar 27,28, wobei die obere Anpresswalze 28 aus mehreren profilartigen auf einer Welle unabhängig voneinander drehbaren Scheiben 28′ (Fig.2a) gebildet ist. Dadurch wird eine annähernd konstante Umfangsgeschwindigkeit der Scheiben 28′ über die gesamte Saugkissen-Arbeitsbreite sichergestellt und eine gleichmässige Anpresskraft über die gesamte geformte Saugkissen-Oberfläche und somit ein einwandfreies Profil der Saugkissen gewährleistet.

Zum Unterschied zur Fig.1 ist in der Ausbildung gemäss der Fig.2 ebenfalls eine bewegbare Hubkammwalze 25 vorgesehen, die jedoch mit einer unterhalb des Siebbandes 23 anliegenden vertikal bewegbaren Hub-Profilwalze 16 derart wirkt, so dass zusammen mit der Hub-Profilwalze 16 das Siebband 23′ (strichliert dargestellt) in den Bereich der über die Breite der Hubkammwalze 15 profilierten Benadelung 14′ anhebbar ist.

Vorzugsweise weist die Benadelung 14′ eine zur Mitte der Hubkammwalze 15 sich vermindernde Höhe der einzelnen Nadeln 14′ auf. Durch das Zusammenwirken der Hubkammwalze 15 und der Hub-Profilwalze 16 können verschiedene Varianten der Saugkissen erzielt werden, so dass dadurch auch deren Profile veränderbar sind. Wie in Fig.2 dargestellt ist die Hub-Profilwalze 16 zusammen mit dem Siebband 23 (strichliert angedeutet) auf die Höhe h angehoben und die Hubkammwalze 15 wird in einer vorbestimmten Position fixiert. Es könnten jedoch sowohl die Hub-Profilwalze 16 als auch die Hubkammwalze 15 gesteuert vertikal zu- und voneinander bewegbar ausgebildet sein. Falls die Hub-Profilwalze 16 unterhalb des Siebbandes 23 verbleibt und die Hubkammwalze mit der Benadelung 24' oder 15' vertikal anheb- und absenkbar ausgebildet sind, werden die Saugkissen in Querrichtung und Längsrichtung (wie in Fig.1) identisch ausgebildet.

Die Fig. 1a und 2a zeigen eine Kammwalze 14 mit einer über deren Breite profilierten Benadelung 14'. Fig.1b zeigt eine Hubkammwalze 15 mit einer über deren Breite gleichmässig hoch verlaufenden Benadelung 15'. In Fig.2b ist eine Hubkammwalze 15 mit einer variablen Höhe der Benadelung 14' dargestellt, die mit der unterhalb des Siebbandes 23 angeordneten Hub-Profilwalze 16 - wie bereits beschrieben- zusammenwirkt.

Fig.2c zeigt im Querschnitt das Anpress-Walzenpaar 27,28, bei welchem die obere Anpresswalze 28 aus mehreren voneinander unabhängig frei drehenden Scheiben 28' besteht. Dadurch wird eine gleichmässige Verfestigung der Saugkissen über die gesamte Oberfläche -ohne das Profil des Saugkissen zu deformieren- gewährleistet.

In Fig.2d sind zwei und teilweise ein drittes endlos verbundenes in Querrichtung 41 und Längsrichtung 42 geformtes Saugkissen 40 dargestellt, wobei zwischen den einzelnen Saugkissen 40 Trennkanten 43 angedeutet sind.

Fig.2e zeigt den Vakuumtisch 18 mit den einzelnen Lagerungen 31, in welche beispielsweise Rollen 32 eingesetzt sind, deren Oberkanten geringfügig über den Vakuumtisch 18 herausragen. Zwischen den Rollen 32 und dem Vakuumtisch 18 sind schlitzförmige Vakuum-Oeffnungen 33 vorgesehen. Anstelle der Rollen könnten auch aus Kugeln gebildete Lagerungen (nicht dargestellt) vorgesehen werden, wobei dann die Vakuumöffnungen die unterschiedlichsten Formen, beispielweise Rundlöcher aufweisen könnten. Die über den Vakuumtisch 18 geringfügig herausragenden Oberkanten der Walzen oder Kugeln 32 gewährleisten einen störungsfreien Lauf des Siebbandes 23 auch bei sehr hoch angelegtem Vakuum.

## Ansprüche

1. Vorrichtung zur Formung von Saugki ssen zum Aufsaugen von Körperflüssigkeit in Hygieneartikeln, insbesondere zur Herstellung körpergerecht geformter Saugkissen für Baby- und Erwachsenen-Windeln, Damenbinden, Slipeinlagen u.dgl., im wesentlichen bestehend aus einem Vakuumtisch (18), über welchen ein endloses, über eine Antriebswalze (20), eine Walze (21) und mindestens eine Spannwalze (22) führbares Siebband (23) angeordnet ist, einer Kammwalze (14) für ein zu bildendes Saugkissen-Profil in Querrichtung des Siebbandes (23), Abwickelvorrichtungen (24, 24') zur Führung einer unteren (25) und einer oberen (25') Abdeck-Papierbahn und einer Dosiereinrichtung (30) für chemische Absorptionsmittel, dadurch gekennzeichnet, dass in Querrichtung des Siebbandes (23) mindestens eine Kammwalze (14) mit je einer über deren Breite unterschiedlich hohen Benadelung (14') und nachfolgend in Längsrichtung (42) der auf dem Siebband (23) endlos aufliegenden Saugkissen (40) eine Hubkammwalze (15) mit einer über deren Breite gleich hohen bzw. unterschiedlich hohen Benadelung (15' bzw.14') angeordnet sind, und zum Verfestigen der Saugkissen (40) ein Anpress-Walzenpaar (27,28) angeordnet ist, wobei die obere Anpresswalze (28) aus mehreren, auf einer Welle frei unabhängig voneinander drehenden Scheibenwalzen (28') und einer unteren glatt ausgebildeten Anpresswalze (27) gebildet ist.

2. Vorrichtung nach Anspruch 1, dadurch gekennzeichnet, dass die vertikal bewegbare Hubkammwalze (15) in das in Querrichtung des Siebbandes (23) gebildete Saugkissen-Profil (44) absenkbar ist.

3. Vorrichtung nach Anspruch 1, dadurch gekennzeichnet, dass die Hubkammwalze (15) mit der in Längsrichtung unterschiedliche Höhen der Benadelung (14') aufweisenden, mit der unterhalb des Siebbandes (23) anliegenden vertikal bewegbaren Hub-Profilwalze (16) derart zusammenwirkt, dass mit der Hub-Profilwalze (16) gleichzeitig das Siebband (23) in den Bereich der Benadelung (14') der Hubkammwalze (15) anhebbar ist.

4. Vorrichtung nach Anspruch 1 und/oder 3, dadurch gekennzeichnet, dass zur Luftwirbeldämpfung die Benadelungen (14',15') der Kammwalzen (14) und/oder der Hubkammwalzen (15) ein verstellbares flach-propellartiges Profil aufweisen.

5. Vorrichtung nach Anspruch 1, dadurch gekennzeichnet, dass zwischen der Vakuumtisch-Oberfläche (18) und dem Siebband (23) im Vakuumtisch (18) gelagerte Rollwalzen (32), und/oder aus Kugeln bestehende Lagerungen derart angeordnet sind, dass zwischen den Lagerungen (31,32) und dem Vakuumtisch (18) Vakuum-Oeffnungen (33) vorgesehen sind.

6. Vorrichtung nach Anspruch 1, dadurch gekennzeichnet, dass die Antriebswalze (20) und die Anpresswalzen (27,28) synchrone Umfangsgeschwindigkeiten aufweisen.

FIG.1a    FIG.1b

14'

14

15'

15

23

23

13

13'

12

19

24'

10

11

25'

30

15

30-3

26

28

30-2

30-1

A    B

14

27

25

29

20    A    B    21

24    17    22    32    17'    17    17

23

FIG.1

FIG. 2a   FIG. 2b   FIG. 2c

FIG. 2

FIG. 2d

FIG. 2e